# EUROPÄISCHE PATENTANMELDUNG

(11) **EP 2 845 710 A1**
(43) Veröffentlichungstag der Anmeldung: **11.03.2015**
(21) Anmeldenummer: 14003050.3
(22) Anmeldetag: 04.09.2014
(51) Int. Cl.: B29C 45/00, A61M 5/32, A61M 5/34

(54) **Verwendung eines Werkzeugs zum Spritzgießen eines Kunststoffkörpers**

(30) Priorität: 06.09.2013 DE 102013014885
(71) Anmelder: Braunform GmbH, 79353 Bahlingen (DE)
(72) Erfinder: Herb, Eckard, 79346 Endingen (DE)
(74) Vertreter: Drobnik, Stefanie

(57) **Zusammenfassung**

Die vorliegende Erfindung bezieht sich auf einen Kunststoffkörper (1) zum Hallen eines nadelförmigen Teils (2) und auf ein Verfahren zu dessen Herstellung. Der Kunststoffkörper (1) weist dabei eine hülsenartige Aufnahme (4) auf, durch die das nadelförmige Teil (2) in den fertigen Kunststoffkörper (1) eingeführt werden kann. Erfindungemäß ist an einen Basiskörper (3) des Kunststoffltörpers (1) ein äußerer zylinderartiger Mantelkörper (5) angeformt, der an seinem vorderen Umfangsrand eine radiale Umstülpung nach innen aufweist. Diese bildet einen inneren zylinderartigen Mantelkörper (6), der die hülsenartige Aufnahme (4) für das nadelförmige Teil (2) im innern definiert.

## Beschreibung

Die Erfindung betrifft einen Kunststoffkörper zum Halten eines nadelförmigen Teils nach dem Oberbegriff des Anspruchs 1; die Erfindung betrifft weiterhin ein Verfahren zum Spritzgießen eines derartigen Kunststoffkörpers zum Halten eines nadelförmigen Teils nach dem Oberbegriff des Anspruchs 5.

Allgemein geht es bei der Erfindung um einen Kunstsloffkörper, der ein nadelförmiges Teil halten soll. Ein besonderes Anwendungsgebiet der Erfindung ist dabei die Kappe einer Spritze, welche die Injektionsnadel halten soll.

Die bekannten Kunststoffkörper zum Halten eines nadelförmigen Teils weisen einen Basiskörper auf. An diesen Basiskörper ist eine hülsenartige Aufnahme angeformt, in die das nadelförmige Teil einführbar ist. Das Problem bei diesen Kunststoffkörpern ist, dass die hülsenartige Aufnahme für das nadelförmige Teil relativ starr ist, so dass das Einführen des nadelförmigen Teils erschwert ist. Insbesondere ist auch die axiale Ausrichtung des nadelförmigen Teils bezüglich des Kunststoffkörpers nicht immer exakt gegeben.

Derartige Kunststoffkörper zum Halten eines nadelförmigen Teils werden üblicherweise im Spritzgießverfahren hergestellt. Dabei wird ein Spritzgießwerkzeug verwendet, das aus zwei Werkzeughälften besteht, die öffenbar sowie schließbar sind. In der Trennfläche zwischen den beiden Werkzeughälften sind Formenhohlräume für die zu spritzenden Kunststoffkörper ausgebildet. Im vorliegenden Fall, bei dem der Kunststoffkörper eine hülsenartige Aufnahme aufweisen soll, ist dem jeweiligen Formenhohlraum ein nadelförmiger Formkern zugeordnet, welcher von dem Kunststoff umspritzt wird und nach dem Erstarren der Schmelze die hülsenartige Aufnahme des Kunststoffkörpers für die Nadel definiert.

Das Problem bei diesem bekannten Spritzgießwerkzeug ist, dass sich der nadelförmige Formkern während des Spritzvorganges aufgrund entstehender Biegemomente durch den Fülldruck der Schmelze sehr leicht verbiegt. Denn der zugeführte viskose Kunststoff trifft einseitig auf diesen nadelförmigen Formkern, da der viskose Kunststoff von einer Seite her dem Werkzeug zugeführt wird. Dies bedeutet, dass der viskose Kunststoff zunächst von einer Seite her auf das auskragende freie Ende des dünnen, nadelförmigen Formkerns trifft. Die Folge davon ist, dass die mit dem nadelförmigen Formkern geschaffene hülsenartige Aufnahme für das nadelförmige Teil aufgrund der Auslenkung des nadelförmigen Formkerns während des Spritzvorganges nicht achsparallel ist.

Der Erfindung liegt die Aufgabe zugrunde, einen Kunststoffkörper zum Halten eines nadelförmigen Teils zu schaffen, bei dem das nadelförmige Teil besser in die hülsenartige Aufnahme einführbar ist; ferner soll ein Verfahren zum Spritzgießen eines Kunststoffkörpers zum Halten eines nadelförmigen Teils geschaffen werden, bei dem die hülsenartige Aufnahme für das nadelförmige Teil exakt achsparallel ausgerichtet ist.

Diese Aufgabe wird durch den Kunststoffkörper zum Halten eines nadelförmigen Teils mit den Merkmalen des Anspruchs 1 gelöst.

Die weitere Aufgabe der Schaffung eines Verfahrens zur Herstellung solcher Kunststoffkörper wird durch das Verfahren mit den Merkmalen des Anspruchs 5 gelöst.

In einer ersten Ausführungsform der Erfindung wird ein Kunststoffkörper zum Halten eines nadelförmigen Teils offenbart, bei dem dieses nadelförmige Teil problemlos in die im Kunststoffkörper ausgebildete hülsenartige Aufnahme einführbar ist, und zwar nachdem der Kunststoffkörper gefertigt worden ist. Dabei wird an einen Basiskörper des Kunststoffkörpers ein äußerer zylinderartiger Mantelkörper angeformt , der an seinem zum (in Benutzungsanordnung) freien Nadelende weisenden vorderen Umfangsrand nach radial innèn und an der Innenseite des äußeren zylinderartigen Mantelkörpers nach unten Richtung Basiskörper fortgeführt wird. Er bildet in der Fortführung quasi einen inneren zylinderartigen Mantelkörper bildet, der die hülsenartige Aufnahme für das nadelförmige Teil im Innern definiert.

Der Kerngedanke des erfindungsgemäßen Kunststoffkörpers besteht also darin, dass die hülsenartige Aufnahme für das nadelförmige Teil nicht in einem in sich massiven Fortsatz des Kunststoffkörpers angeordnet ist, sondern dass durch die Umbördelung oder Umstülpung des zylinderartigen Mantelkörpers das System eine definierte innere Elastizität besitzt, die das Einführen des nadelförmigen Teils vereinfacht. Die Begriffe "Umbördelung" oder "Umstülpung" sind dabei nur zur bildhaften Darstellung zu verstehen, denn, wie sich auch aus dem Verfahren ergibt, das Teil wird im Spritzguss hergestellt und die äußeren und inneren Mantelkörper werden einstückig gegossen. Die innere, umgestülpte Kunststoffhülse der hülsenförmigen Aufnahme ist leicht flexibel innerhalb der äußeren Kunststoffhülse gelagert, resp. positioniert. Die beiden zylinderartigen Mantelkörper weisen dabei zwischen sich einen Abstand auf, wobei bezüglich der Umbördelung oder Umstülpung - im Schnitt gesehen - die beiden Schenkel einen spitzen Winkel einschließen. Anders gesagt: Die beiden Wandungen, die den inneren und äußeren zylinderartigen Mantelkörper bilden, formen eine im Querschnitt hohlkegelartige Vertiefung.

Gemäß der Weiterbildung in Anspruch 2 erstreckt sich der innere zylinderartige Mantelkörper im Wesentlichen bis zur Basis des äußeren, zylinderförmigen Mantelkörpers. Dadurch ist für das nadelförmige Teil in der hülsenartigen Aufnahme des inneren, zylinderartigen Metallkörpers ein einwandfreier Halt gewährleistet.

Um das Einführen des nadelförmigen Teils in die hülsenartige Aufnahme zu verbessern, schlägt die Weiterbildung gemäß Anspruch 3 vor, dass der innere, zylinderartige Mantelkörper im Einführungsbereich für dieses nadelförmige Teil konisch ausgebildet ist.

Vorzugsweise ist gemäß der Weiterbildung in Anspruch 4 der Kunststoffkörper im Spritzgießverfahren hergestellt.

Gemäß einer Ausführungsform des erfindungsgemäßen Verfahrens zum Spritzgießen eines Kunststoffkörpers, der zum Halten eines nadelförmigen Teils geschaffen ist und bei dem die hülsenartige Aufnahme für das nadelförmige Teil exakt achsparallel ausgerichtet ist, wird der nadelförmige Formkern nicht ausschließlich von einer Seite her mit dem zugeführten viskosen Kunststoffmaterial beaufschlagt, sondern er wird um seinen gesamten Umfang herum gleichmäßig mit dem zugeführten viskosen Kunststoffmaterial beaufschlagt. Erreicht wird dies dadurch, dass zunächst sich der zugeführte viskose Kunststoff im äußeren, zylinderartigen Mantelkörper gleichmäßig verteilt, um anschließend in dieser Gleichverteilung hin zum nadelförmigen Formkern zu fließen. Dadurch erfolgt eine gleichmäßige umfangsmäßige Druckbeaufschlagung durch den zugeführten viskosen Kunststoff. Der wesentliche Vorteil dadurch ist, dass es keine Verformungen in der hülsenartigen Aufnahme für das einzuführende nadelförmige Teil gibt.

Ein Ausführungsbeispiel eines erfindungsgemäßen Kunststoffkörpers zum Einführen eines nadelförmigen Teils in Form einer Injektionsnadel und das Spritzgießverfahren eines derartigen Kunststoffkörpers werden nachfolgend beispielhaft zum besseren Verständnis des Gegenstands an Hand der Figuren beschrieben. In diesen zeigt:
- **Fig. 1a**: eine perspektivische Ansicht des Kunststoffkörpers mit eingeführtem nadelförmigen Teil;
- **Fig. 1b**: eine Längsschnittdarstellung durch den Kunststoffkörper mit nadelförmigem Teil in **Fig. 1a****;**
- **Fig. 2a**: eine perspektivische Ansicht des Kunststoffkörpers ohne nadelförmiges Teil;

- **Fig. 2b**: eine Längsschnittdarstellung durch den Kunststoffkörper ohne nadelförmiges Teil in **Fig. 2a****;**
- **Fig. 3**: eine Darstellung des Spritzgießvorganges in 10 Schritten zum Herstellen eines derartigen Kunststoffkörpers.

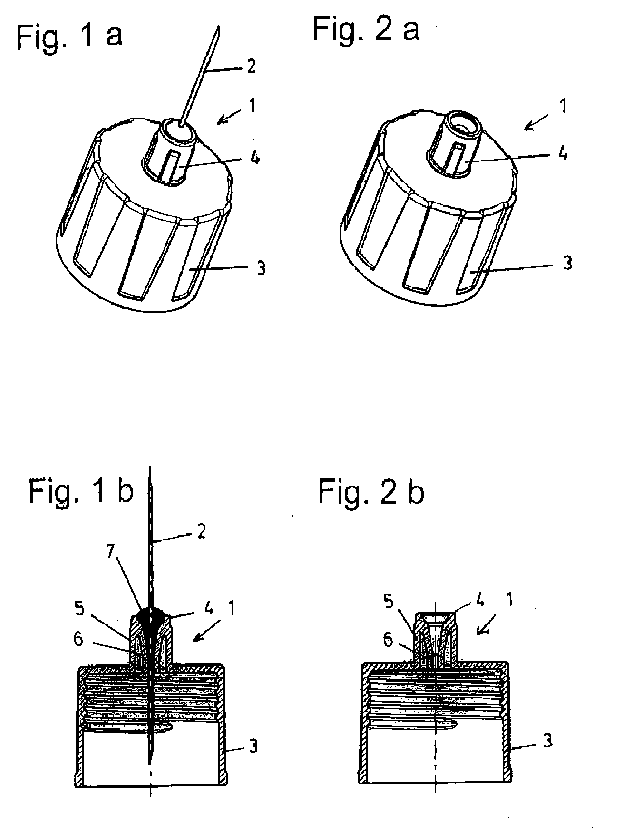

Fig. 1a und 1b zeigen einen Kunststoffkörper 1 in Form einer Injektionskappe mit einem eingeführten nadelförmigen Teil 2 in Form einer Injektionsnadel. Die Kappe dient somit als Halterung für eine Injektionsnadel.

Der Kunststoffkörper 1 weist zunächst einen Basiskörper 3 in Form einer Schraubkappe auf. An diesem Basiskörper 3 ist eine hülsenartige Aufnahme 4 für das nadelförmige Teil 2 angeformt.

Konkret weist diese hülsenartige Aufnahme 4 zunächst einen äußeren, zylinderartigen Mantelkörper 5 auf. Dieser ist im vorderen Stirnbereich radial nach innen sowie zurück zurückge-führt; optisch im Sinne einer Umbördelung oder Umstülpung, und definiert in diesem inneren Bereich einen inneren, zylinderartigen Mantelkörper 6. Dieser innere, zylinderartige Mantelkörper 6 reicht im Wesentlichen bis zur Basis des äußeren, zylinderartigen Mantelkörpers 5. Außerdem ist er konisch ausgebildet. Wie insbesondere in **Fig. 1b** erkennbar ist, ist das nadelförmige Teil 2 in diesen inneren, konischen Mantelkörper 6 eingeführt und mittels eines Klebemittels 7 gesichert.

Die **Fig. 2a** und **2b** zeigen nochmal die gleiche Situation, jedoch ohne das nadelförmige Teil 2.

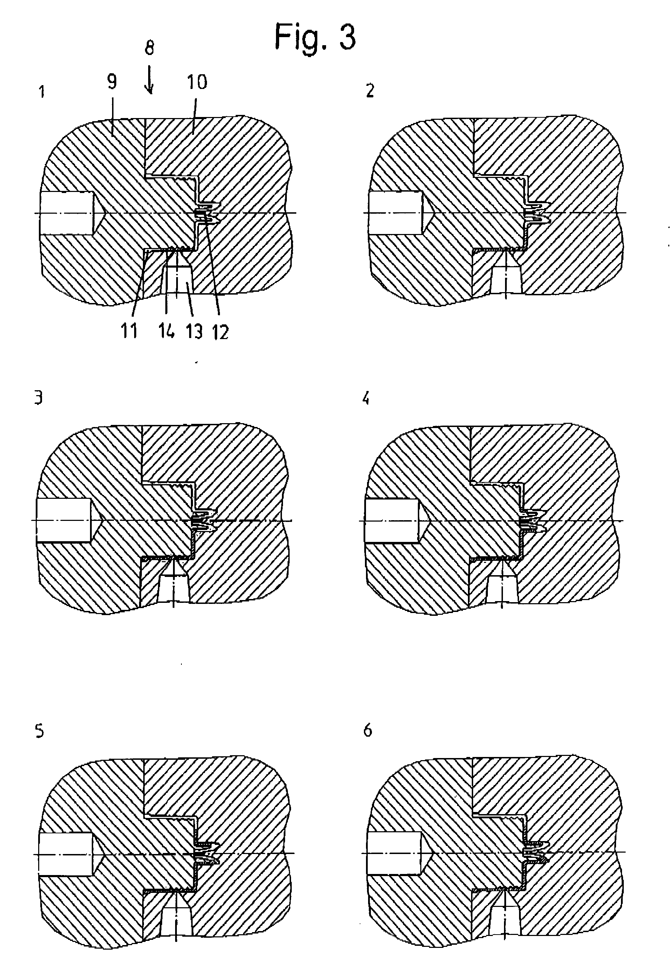

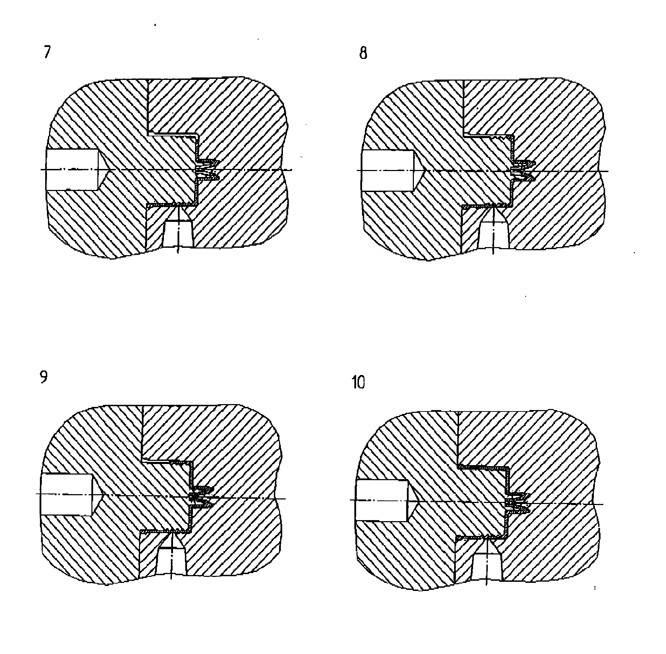

**Fig. 3** zeigt in 10 Schritten, wie sich der Kunststoffkörper 1 im Spritzgießverfahren herstellen läßt:

Schritt 1 zeigt die Ausgangssituation mit einem Spritzgießwerkzeug 8, dessen beide Werkzeughälften 9, 10 geschlossen sind und zwischen sich wenigstens einen einzigen, jedoch vorzugsweise mehrere Formenhohlräume 11 für den Kunststoffkörper 1 definieren. Dabei weist die in der Zeichnung rechte Werkzeughälfte 10 noch einen nadelförmigen Formkern 12 auf. Außerdem weist die in der Zeichnung rechte Werkzeughälfte 10 eine Zuführung 13 für viskosen Kunststoff auf.

Im Schritt 1 ist erkennbar, dass über die Zuführung 13 bereits viskoser Kunststoff 14 in den Formenhohlraum 11 eingedrungen ist, und zwar in dem Bereich, welcher später den Basis-körper 3 des Kunststoffkörpers 1 definiert.

Die Schritte 2 bis 6 zeigen, wie der viskose Kunststoff 14 allmählich vordringt und schließlich den Formenhohlraum 11 im Bereich des äußeren, zylinderartigen Mantelkörpers 5 des Kunststoffkörpers 1 um den gesamten Umfang herum ausfüllt.

Die Schritte 7 bis 9 zeigen, wie der viskose Kunststoff die Umstülpung oder -bördelung zwischen den beiden - späteren - äußeren sowie inneren, zylinderartigen Mantelkörpern 5, 6 des Formenhohlraumes 11 passiert und dabei gleichmäßig um den gesamten Umfang herum schließlich bis zum Vorderende des nadelförmigen Formkerns 12 fließt. Durch dieses gleichmäßige Umfließen des nadelförmigen Formkerns 12 um den gesamten Umfang herum wirken auf diesen keine Biegemomente, so dass er seine vorgegebene axiale Position exakt einhält.

Der Schritt 10 schließlich zeigt noch die Situation, wenn der komplette Formenhohlraum 11 im Bereich des späteren Basiskörpers 3 des Kunststoffkörpers 1 mit dem viskosen Kunststoff 14 ausgefüllt ist.

Nach dem Erkalten des viskosen Kunststoffs 14 werden die beiden Werkzeughälften 9, 10 des Spritzgießwerkzeugs 8 geöffnet und der Kunststoffkörper 1 entnommen.

### BEZUGSZEICHENLISTE

- 1: Kunststoffkörper
- 2: nadelförmiges Teil
- 3: Basiskörper
- 4: hülsenartige Aufnahme
- 5: äußerer zylinderartiger Mantelkörper
- 6: innerer zylinderartiger Mantelkörper
- 7: Klebemittel
- 8: Spritzgießwerkzeug
- 9, 10: Werkzeughälften
- 11: Formenhohlraum
- 12: nadelförmiger Formkern
- 13: Zuführung
- 14: viskoser Kunststoff

## Patentansprüche

1. Kunststoffkörper (1) zum Halten eines nadelförmigen Teils (2),
wobei der Kunststoffkörper (1) eine hülsenartige Aufnahme (4) aufweist, durch die das nadelförmige Teil (2) in den fertigen Kunsistoffkörper (1) einführbar ist,
**dadurch gekennzeichnet,**
**dass** an einen Basiskörper (3) des Kunststoffkörpers (1) ein äußerer zylinderartiger Mantelkörper (5) angeformt ist,
der an seinem vorderen Umfangsrand eine radiale Umstülpung nach innen aufweist, die einen inneren zylinderartigen Mantelkörper (6) bildet, der die hülsenartige Aufnahme (4) für das nadelförmige Teil (2) im Innern definiert.

2. Kunststoffkörper nach dem vorhergehenden Anspruch,
**dadurch gekennzeichnet,**
**dass** sich der innere zylinderartige Mantelkörper (6) im Wesentlichen bis zur Basis des äußeren zylinderartigen Mantelkörpers (5) erstreckt.

3. Kunststoffkörper nach einem der vorhergehenden Ansprüche,
**dadurch gekennzeichnet,**
**dass** der innere zylinderartige Mantelkörper (6) im Einführungsbereich für das nadelförmige Teil (2) konisch ausgebildet ist.

4. Kunststoffkörper nach einem der vorhergehenden Ansprüche,
**dadurch gekennzeichnet,**
**dass** der Kunststoffkörper (1) ein spritzgegossener Kunststoffkörper (1) ist.

5. Verfahren zum Spritzgießen eines Kunststoffkörpers (1) zum Halten eines nadelförmigen Teils nach einem der Ansprüche 1 bis 4,
unter Verwendung eines Spritzgießwerkzeugs (8) bestehend aus zwei öffen- und schließbaren Werkzeughälften (9, 10),
zwischen denen Formenhohlräume (11) für die zu spritzenden Kunststoffkörper (1) ausgebildet sind und
wobei den Formenhohlräumen (11) jeweils ein nadelförmiger Formkern (12) zur Ausbildung der hülsenartigen Aufnahme (4) des Kunststoffkörpers (1) zugeordnet ist, **dadurch gekennzeichnet,**
**dass** zunächst sich der zugeführte viskose Kunststoff (14) um den gesamten Umfang des Bereichs des Formenhohlraums (11) für den späteren äußeren zylinderartigen Mantelkörper (5) herum verteilt und
**dass** anschließend sich der viskose Kunststoff (14) in den Bereich des Formenhohlraums (11) für den späteren inneren zylinderartigen Mantelkörper (6) des Kunststoffkörpers (1) fließt.
